# EUROPEAN PATENT SPECIFICATION

(11) **EP 3 893 948 B1**
(45) Date of publication and mention of the grant of the patent: **06.12.2023**
(21) Application number: 19836739.3
(22) Date of filing: 12.12.2019
(51) Int. Cl.: A61L 15/60, A61L 15/18, A61L 15/20, A61L 15/46, A61F 13/84

(54) **ODOR-INHIBITING COMPOSITIONS AND HYGIENIC ARTICLES INCORPORATING THE SAME**
GERUCHSHEMMENDE ZUSAMMENSETZUNGEN UND HYGIENEARTIKEL DAMIT
COMPOSITIONS D'INHIBITION DES ODEURS ET ARTICLES HYGIÉNIQUES INCORPORANT CELLES-CI

(30) Priority: 13.12.2018 US 201862779453 P
(43) Date of publication of application: 20.10.2021
(73) Proprietor: BASF SE, 67056 Ludwigshafen am Rhein (DE)
(72) Inventor: HOELLER, Olaf, 67056 Ludwigshafen (DE); RODRIGUEZ, David J, Tarrytown, NY 10591 (US); LEPTICH, Joseph, Florham Park, NJ 07932 (US); WOLFE, Michaela P., Florham Park, NJ 07932 (US); MITCHELL, Michael, Charlotte, NC 28273 (US)
(74) Representative: BASF IP Association
(86) International application number: PCT/US2019/066000
(87) International publication number: WO 2020/123821

(56) References cited:
- EP-A1- 1 034 801
- WO-A1-00/65911
- WO-A1-2006/033477
- WO-A1-2015/132267
- US-A1- 2004 116 882
- US-A1- 2011 182 845
- US-A1- 2015 306 272

## Description

### BACKGROUND

Water-absorbing polymer particles, also referred to as superabsorbents, are used in odor-inhibiting compositions for diapers, tampons, sanitary napkins, and other hygienic articles. The properties of the water-absorbing polymer particles can be adjusted in various ways, such as by adjusting the amount of crosslinking within the polymer matrix of the particles. However, while increasing the amounts of certain components in the composition may improve the odor-inhibiting ability of the composition, this often results in a tradeoff with other desirable properties of the composition, such as centrifuge retention capacity (CRC) and absorbance against pressure (AAP) Patent application WO2006/033477 discloses a particulate water-absorbing agent which not only ensures the many conventional physical properties (absorption rate, centrifuge retention capacity, absorbency against pressure, particle size distribution etc.), but also prevents odor which is generated after the resin is swollen by absorption.The water absorbent agent therefor does not cause odor in actual use.

### SUMMARY OF THE DISCLOSURE

The odor-inhibiting composition as claimed, comprises: water-absorbing polymer particles; a first odor control agent comprising a chelation agent; and a second odor control agent, wherein the chelation agent is selected from a group consisting of: ethylenediaminetetraacetic acid (EDTA), nitrilotris(methylene)triphosphonic acid, sodium triphosphate pentabase, N-carboxymethylated polethyleneimine, and 1-hydroxyethane 1,1-diphosphonic acid, or salts thereof; and the second odor control agent is a membrane lysing compound selected from a group consisting of: 1,2-decanediol, cetrimonium chloride, cocamidopropyl betaine, poly(t-butylaminoethylmethacrylate), behentrimonium, and 1,2-pentanediol.

In one aspect of the present disclosure, an odor-inhibiting composition comprises: water-absorbing polymer particles; a first odor control agent comprising a chelation agent, wherein the first odor control agent is present from about 0.25 wt.% to about 1.0 wt.% based on a total weight of the water-absorbing polymer particles; and a second odor control agent present from about 0.25 wt.% to about 2.0 wt.% based on the total weight of the water-absorbing polymer particles.

In one embodiment, the second odor control agent is present from about 0.25 wt.% to about 1.0 wt.% based on the total weight of the water-absorbing polymer particles.

In one embodiment, the water-absorbing polymer particles comprise crosslinked polyacrylic acid.

In one embodiment, wherein the water-absorbing polymer particles are polymerized in the presence of one or more of the first odor control agent or the second odor control agent.

The chelation agent is selected from a group consisting of: ethylenediaminetetraacetic acid (EDTA), nitrilotris(methylene)triphosphonic acid, sodium triphosphate pentabasic, N-carboxymethylated polyethyleneimine, and 1-hydroxyethane 1,1-diphosphonic acid or salts thereof.

The second odor control agent is a membrane lysing compound selected from a group consisting of: 1,2-decanediol, cetrimonium chloride, cocamidopropyl betaine, poly(t-butylaminoethylmethacrylate), behentrimonium, and 1,2-pentanediol.

In one embodiment, the second odor control agent comprises 1,2-decanediol or cetrimonium chloride.

In one embodiment, the composition exhibits an absorbance against pressure (AAP) of at least 20 g/g.

In one embodiment, the composition exhibits a centrifuge retention capacity (CRC) of at least 30 g/g.

In one embodiment, the composition exhibits an ammonia breakthrough time of greater than 45 hours at about 23 °C.

In one embodiment, the composition comprises a third odor control agent, wherein the third odor control agent is a different compound than the first odor control agent and the second odor control agent. In one embodiment, the third odor control agent comprises zinc peroxide. In one embodiment, the first odor control agent comprises 1-hydroxyethane 1,1-diphosphonic acid or salts thereof, wherein the second odor control agent comprises cetrimonium chloride, and wherein the third odor control agent comprises zinc peroxide.

In another aspect of the present disclosure, a method of producing an odor-inhibiting composition comprises: infusing water-absorbing polymer particles with a first odor control agent comprising a chelation agent; mixing the infused water-absorbing polymer particles with a second odor control agent, as disclosed in claim 8, wherein the first odor control agent is present from about 0.25 wt.% to about 1.0 wt.% based on a total weight of the water-absorbing polymer particles, and wherein the second odor control agent is present from about 0.25 wt.% to about 2.0 wt.% based on the total weight of the composition.

In one embodiment, the second odor control agent is present from about 0.25 wt.% to about 1.0 wt.% based on the total weight of the water-absorbing polymer particles.

In one embodiment, infusing water-absorbing polymer particles with the first odor control agent comprises polymerizing a monomer solution in the presence of the first odor control agent.

The chelation agent is selected from a group consisting of: EDTA, nitrilotris(methylene)triphosphonic acid, sodium triphosphate pentabasic, N-carboxymethylated polyethyleneimine, and 1-hydroxyethane 1,1-diphosphonic acid or salts thereof.

The second odor control agent is a membrane lysing compound selected from a group consisting of: 1,2-decanediol, cetrimonium chloride, cocamidopropyl betaine, poly(t-butylaminoethylmethacrylate), behentrimonium, and 1,2-pentanediol.

In one embodiment, the second odor control agent comprises 1,2-decanediol or cetrimonium chloride.

In one embodiment exhibits an absorbance against pressure (AAP) of at least 20 g/g.

In one embodiment the composition exhibits a centrifuge retention capacity (CRC) of at least 30 g/g.

In one embodiment the composition exhibits an ammonia breakthrough time of greater than 45 hours at about 23 °C.

In another aspect of the present disclosure, a hygienic article comprises an odor-inhibiting composition, the composition comprising: water-absorbing polymer particles; a first odor control agent comprising a chelation agent, and a second control agent, as disclosed in claim 12, wherein the first odor control agent is present from about 0.25 wt.% to about 1.0 wt.% based on a total weight of the water-absorbing polymer particles; and a second odor control agent present from about 0.25 wt.% to about 2.0 wt.% based on the total weight of the water-absorbing polymer particles.

In one embodiment, the hygienic article is selected from a group consisting of: a diaper, a diaper pad, a feminine hygienic article, an incontinence article, a bed pad, a nursing pad, animal litter, an animal training pad, and a pet diaper.

"Centrifuge retention capacity," as referred to herein, is determined according to the standard test method Nonwovens Standard Procedures (NWSP) 241.0.R2 "Gravimetric Determination of the Fluid Retention Capacity in Saline Solution after Centrifugation."

"Absorbance against pressure," as referred to herein, is determined according to the standard test method NWSP 242.0.R2 "Gravimetric Determination of Absorption against Pressure."

Also as used herein, the units of "wt.%" refer to the weight-to-weight ratio of a particular material with respect to a total weight of a reference material. For example, if composition C weighs 10 grams in total and is comprised of 5 grams of compound A, 2 grams of compound B, and 3 grams of other materials, compound A is said to be present at 50 wt.% based on the total weight of composition C.

Also as used herein, the term "particles," as used herein, refers to a collection of discrete portions of a material each having a largest dimension ranging from 0.1 µm to 50 mm. The size ranges disclosed herein can be mean/average or median size, unless otherwise stated. It is noted also that particles need not be spherical, but may be in a form of cubes, cylinders, discs, or any other suitable shape as would be appreciated by one of ordinary skill in the art. A "granule" may be a type of particle.

Also as used herein, the term "about," as used in connection with a measured quantity, refers to the normal variations in that measured quantity, as expected by the skilled artisan making the measurement and exercising a level of care commensurate with the objective of measurement and the precision of the measuring equipment (e.g., within ± 1% of the quantity stated).

### DETAILED DESCRIPTION

The embodiments described herein relate to odor-inhibiting compositions and articles incorporating odor-inhibiting compositions, such as hygienic articles. More specifically, the embodiments relate to multimodal odor-inhibiting compositions that utilize a superabsorbent (i.e., water-absorbing polymer particles) in combination with two or more different odor control agents.

The addition of odor control agents as add-ons normally reduces absorbency properties of superabsorbents, such as centrifuge retention capacity (CRC) and absorbance against pressure (AAP). Therefore, odor control agents are normally kept to a minimum, which reduces the overall effectiveness of the odor control agent.

In accordance with the embodiments herein, the use of a binary mixture of odor control agents with more than one mode of action allows for reduced impact on absorbency properties with increased efficacy of odor control. As used herein, "odor control" or "odor inhibition" refers to the time until generation of ammonia from urea due to bacterial action. It has been found that an addition of two or more odor control agents to, e.g., a superabsorbent or an absorbing structure of a hygienic article increases the time until ammonia odor generation. The lower concentration of each odor control agent allows for the add-on level used to reach high odor control efficacy to be present in low enough amounts that there is little or no impact on absorbency properties, such as CRC and AAP. The odor control agents can be added to a superabsorbent, but also to the nonwoven, fluff, or adhesive components of the diaper.

The odor-inhibiting composition comprises water-absorbing polymer particles, and two, or more odor control agents. In some embodiments, the composition includes three odor control agents.

In some embodiments, the water-absorbing polymer particles are produced, for example, by polymerizing a monomer solution or suspension comprising at least one ethylenically unsaturated monomer which bears acid groups and may be at least partly neutralized, at least one crosslinker, at least one initiator, optionally at least one ethylenically unsaturated monomer that is copolymerizable with the other aforementioned monomer, and optionally one or more water-soluble polymers. Suitable monomers may include, for example, ethylenically unsaturated carboxylic acids (e.g., acrylic acid, methacrylic acid, itaconic acid, etc.) and ethylenically unsaturated sulfonic acids (e.g., styrenesulfonic acid and 2-acrylamido-2-methylpropanesulfonic acid). In some embodiments, the water-absorbing polymer particles comprise crosslinked polyacrylic acid.

In some embodiments, the water-absorbing polymer particles may be produced as described in U.S. Patent No. 9,433697. Suitable water-absorbing polymer particles may include, for example, HySorb^{®} T6600, HySorb^{®} B7055, and HySorb^{®} B7065 (BASF SE, Ludwigshafen, Germany).

In some embodiments, the water-absorbing polymer particles, when dry, are in powder form and are characterized by a mean particle size of at least about 200 µm, from about 200 µm to about 800 µm, from about 250 µm to about 750 µm, from about 300 µm to about 600 µm, or from about 300 µm to about 500 µm.

Regarding odor control agents, the chelation agents are selected from a group consisting of: ethylenediaminetetraacetic acid (EDTA), nitrilotris(methylene)triphosphonic acid solution, sodium triphosphate pentabasic, N-carboxymethylated polyethyleneimine (e.g., Trilon^{®} P liquid available from BASF), and 1-hydroxyethane 1,1-diphosphonic acid or salts thereof.

The membrane lysing compounds are selected from a group consisting of: 1,2-decanediol, cetrimonium chloride, cocamidopropyl betaine, poly(t-butylaminoethylmethacrylate), behentrimonium, and 1,2-pentanediol.

In one advantageous embodiment, a chelation agent is used as a first odor control agent in combination with 1,2-decanediol as a second odor control agent. In another advantageous embodiment, a chelation agent is used as a first odor control agent in combination with cetrimonium chloride as a second odor control agent.

The odor-inhibiting composition may include one or more food or cosmetic preservatives in addition to the odor control agents described. Such food of cosmetic preservatives may include, but are not limited to, benzoic acid, sodium benzoate, benzyl alcohol, cetylpyridinium chloride, potassium sorbate, sorbitan caprylate, phenoxyethanol, chlorphenesin, parabens (e.g., propylparaben), ethyl lauroyl arginate, antimicrobial agents (e.g., Nipaguard^{®} brand agents available from Clariant), DMDM hydantoin, and methylisothiazolinone.

The odor-inhibiting composition comprises a first odor control agent (which may be referred to as a primary odor control agent) and a second odor control agent (which may be referred to as an auxiliary odor control agent). The primary odor control agent comprises a chelation agent as disclosed in claim 1. The primary odor control agent may be present from about 0.05 wt.% to about 2.0 wt.% based on a total weight of the water-absorbing polymer particles. In some embodiments, the primary odor control agent is present at about 0.05 wt.%, about 0.10 wt.%, about 0.15 wt.%, about 0.20 wt.%, about 0.25 wt.%, about 0.30 wt.%, about 0.35 wt.%, about 0.40 wt.%, about 0.45 wt.%, about 0.50 wt.%, about 0.55 wt.%, about 0.60 wt.%, about 0.65 wt.%, about 0.70 wt.%, about 0.75 wt.%, about 0.80 wt.%, about 0.85 wt.%, about 0.90 wt.%, about 0.95 wt.%, about 1.00 wt.%, about 1.05 wt.%, about 1.10 wt.%, about 1.15 wt.%, about 1.20 wt.%, about 1.25 wt.%, about 1.30 wt.%, about 1.35 wt.%, about 1.40 wt.%, about 1.45 wt.%, about 1.50 wt.%, about 1.55 wt.%, about 1.60 wt.%, about 1.65 wt.%, about 1.70 wt.%, about 1.75 wt.%, about 1.80 wt.%, about 1.85 wt.%, about 1.90 wt.%, about 1.95 wt.%, about 2.00 wt.%, or within any range defined therebetween (e.g., about 0.50 wt.% to about 1.00 wt.%).

In some embodiments, the auxiliary odor control agent comprises 1,2-decanediol, cetrimonium chloride, or zinc peroxide. The auxiliary odor control agent may be present from about 0.05 wt.% to about 2.0 wt.% based on a total weight of the water absorbing-particles. In some embodiments, the primary odor control agent is present at about 0.05 wt.%, about 0.10 wt.%, about 0.15 wt.%, about 0.20 wt.%, about 0.25 wt.%, about 0.30 wt.%, about 0.35 wt.%, about 0.40 wt.%, about 0.45 wt.%, about 0.50 wt.%, about 0.55 wt.%, about 0.60 wt.%, about 0.65 wt.%, about 0.70 wt.%, about 0.75 wt.%, about 0.80 wt.%, about 0.85 wt.%, about 0.90 wt.%, about 0.95 wt.%, about 1.00 wt.%, about 1.05 wt.%, about 1.10 wt.%, about 1.15 wt.%, about 1.20 wt.%, about 1.25 wt.%, about 1.30 wt.%, about 1.35 wt.%, about 1.40 wt.%, about 1.45 wt.%, about 1.50 wt.%, about 1.55 wt.%, about 1.60 wt.%, about 1.65 wt.%, about 1.70 wt.%, about 1.75 wt.%, about 1.80 wt.%, about 1.85 wt.%, about 1.90 wt.%, about 1.95 wt.%, about 2.00 wt.%, or within any range defined therebetween (e.g., about 0.50 wt.% to about 1.00 wt.%).

In some embodiments, the primary and auxiliary odor control agents are loaded onto the water-absorbing polymer particles. In some embodiments, one or more of the primary or auxiliary odor control agents is infused into the water-absorbing polymer particles. For example, the primary odor control agent (a chelation agent) may be infused in the water-absorbing polymer particles by polymerizing the water-absorbing polymer particles in the presence of the primary odor control agent.

In some embodiments, the odor-inhibiting composition exhibits a CRC of at least about 30.0 g/g, at least about 32.0 g/g, or from about 32.0 g/g to about 35.0 g/g. In some embodiments, the odor-inhibiting composition exhibits a CRC of about 30.0 g/g, about 30.5 g/g, about 31.0 g/g, about 31.5 g/g, about 32.0 g/g, about 32.5 g/g, about 33.0 g/g, about 33.5 g/g, about 34.0 g/g, about 34.5 g/g, about 35.0 g/g, about 35.5 g/g, about 36.0 g/g, about 36.5 g/g, about 37.0 g/g, about 37.5 g/g, about 38.0 g/g, about 38.5 g/g, about 39.0 g/g, about 39.5 g/g, about 40.0 g/g, or within any range defined therebetween (e.g., about 30.0 g to about 35.0 g/g).

In some embodiments, the odor-inhibiting composition exhibits an AAP of at least 20 g/g. In some embodiments, the odor-inhibiting composition exhibits an AAP of about 20.0 g/g, about 20.5 g/g, about 21.0 g/g, about 21.5 g/g, about 22.0 g/g, about 22.5 g/g, about 23.0 g/g, about 23.5 g/g, about 24.0 g/g, about 24.5 g/g, about 25.0 g/g, about 25.5 g/g, about 26.0 g/g, about 26.5 g/g, about 27.0 g/g, about 27.5 g/g, about 28.0 g/g, about 28.5 g/g, about 29.0 g/g, about 29.5 g/g, about 30.0 g/g, or within any range defined therebetween (e.g., about 20.0 g to about 25.0 g/g).

In some embodiments, there is no statistically significant difference in CRC between an odor-inhibiting composition that utilizes primary and auxiliary odor control agents, as described, and a similar odor-inhibiting composition that utilizes a primary odor control agent without an auxiliary odor control agent.

In some embodiments, a decrease in AAP of an odor-inhibiting composition that utilizes primary and auxiliary odor control agents, as described, compared to the AAP of a similar odor-inhibiting composition that utilizes a primary odor control agent without an auxiliary odor control agent is less than about 3 g/g, less than about 2 g/g, or less than about 1 g/g.

In some embodiments, the odor-inhibiting composition is incorporated into a hygienic article, such as a diaper, a diaper pad, a feminine hygienic article, an incontinence article, an animal training pad, and a pet diaper. Such hygienic articles typically comprise a water-impermeable backside, a water-permeable topside, and an absorbent material disposed therebetween. The absorbent material may comprise the odor-inhibiting composition described herein as well as other absorbent components. The hygienic article may also be in the form of granules, such as an absorbent animal litter.

### ILLUSTRATIVE EXAMPLES

The following examples are set forth to assist in understanding the disclosed embodiments and should not be construed as specifically limiting the embodiments described and claimed herein.

The measurements of the following examples were, unless stated otherwise, carried out at an ambient temperature of 23 ± 2° C and a relative air humidity of 50 ± 10%. The water-absorbing polymer particles were mixed thoroughly before performing any measurement.

The following method was used for each of the following examples to determine bacterial-induced ammonia release. Brain heart infusion (BHI) medium was inoculated to OD = 0.1 with Proteus mirabilis ATCC 14153, and incubated in a 10 mL test tube at 37 °C and 220 rpm for 15 hours. The resulting cultures had a cell density of about 10⁹ CFU/mL (OD = 2.0-2.5). Synthetic urine was prepared from 16.7 g/L glucose (sterile-filtered), 50 g/L of urea (sterile-filtered), 9.0 g/L of sodium chloride, 4.0 g/L potassium sulfate, 2.5 g/L ammonia sulfate, 0.8 g/L calcium acetate, 0.7g/L magnesium sulfate, 0.5 g/L yeast extract, 1 g/L of peptone from meat, and 1 g/L of meat extract, with the mixture being autoclaved prior to the addition of the sterile-filtered glucose and urea solutions. 125 mL polypropylene histology beakers were autoclaved, and 1 g of water-absorbing polymer particles was added. Then, 500 mL of synthetic urine were inoculated with 50 µL of bacterial strain solution corresponding to a total concentration of approximately 10⁶ CFU/mL, and 32 mL of which was mixed with the water-absorbing polymer particles. A lid provided with a diffusion testing tube (Drägerwerk AG & Co. KGaA; Lubeck; Germany; Dräger-Tube^{®} Ammonia 20/a-D; Art. No. 8101301) was screwed on immediately after. The evolution of ammonia was observed at 37 °C for greater than 48 hours or at room temperature (about 23 °C) for greater than 70 hours.

As used in the description that follows, "Compound A" refers to di-sodium salt of 1-hydroxyethane 1,1-diphosphonic acid.

### Comparative Example 1

In a 5L Lödige plowshare mixer, 1 kg of HySorb^{®} T6600 superabsorbent particles (which had been dried at 120 °C for 3 hours in a convection oven) was mixed at 265 rpm. While mixing, a solution of 5 parts of 1,2-decanediol (Aldrich Chemical Co.) dissolved in 30 parts 2-propanol was added. After mixing for 5 minutes, the treated superabsorbent was discharged and dried in an explosion-proof oven for 1 hour at 100 °C. This yielded at total of 0.5 wt.% 1,2-decanediol on the HySorb^{®} T6600 (i.e., a 0.5% ratio of the 1,2-decanediol weight to the HySorb^{®} T6600 weight).

### Comparative Example 2

The procedure of Comparative Example 1 was repeated with a solution of 5 parts EDTA-disodium salt (Aldrich) in 30 parts water instead of the 1,2-decanediol solution. This yielded a total of 0.5 wt.% EDTA-disodium salt on the HySorb^{®} T6600.

### Comparative Example 3

The procedure of Comparative Example 1 was repeated with a solution of 5.6 parts of Compound A in 30 parts water instead of the 1,2-decanediol solution. This yielded a total of 0.5 wt.% of Compound A on the HySorb^{®} T6600.

### Comparative Example 4

The procedure of Comparative Example 1 was repeated with a solution of 17.2 parts of cetrimonium chloride (29% solution in water, MakingCosmetics Inc.) in 17.7 parts water instead of the 1-2-decanediol solution. This yielded a total of 0.5 wt.% pure cetrimonium chloride on the HySorb^{®} T6600.

HySorb^{®} T6600 was produced with 0.35 wt.% Compound A in the polymerization to create a HySorb^{®} T6600-Compound A infused sample (commercial product, available from BASF Corp.).

### Comparative Example 6

HySorb^{®} T6600 was used without any treatment (commercial product, available from BASF Corp.).

### Comparative Example 7

HySorb^{®} T6600OC was used without any treatment (commercial product, available from BASF Corp.).

Results of the comparative examples are shown in Table 1. The ammonia breakthrough time (BTT) corresponds to room temperature incubation.

**Table 1: Comparative examples**

| **Sample** | **Agent** | **Agent Cone. (wt.%)** | **CRC (g/g)** | **AAP (g/g)** | **NH₃ BTT (hours)** |
|---|---|---|---|---|---|
| Comparative Example 6 | None | N/A | 33.6 | 23.2 | 15.7 |
| Comparative Example 7 | Zinc peroxide | 0.30 | 34.7 | 21.5 | 50.0 |
| Comparative Example 5 | Compound A | 0.35 | 33.9 | 23.7 | 19.0 |
| Comparative Example 2 | EDTA-disodium salt | 0.50 | 35.8 | 22.1 | 32.0 |
| Comparative Example 3 | Compound A | 0.50 | 35.7 | 22.2 | 28.7 |
| Comparative Example 1 | 1,2-decanediol | 0.50 | 36.0 | 19.9 | 18.0 |
| Comparative Example 4 | cetrimonium chloride | 0.50 | 36.2 | 20.1 | 49.7 |

The inventive examples are now described.

### Example 1

In a 5L Lödige plowshare mixer, 1 kg of HySorb T6600 superabsorbent (infused with 0.35 wt.% Compound A) was mixed at 265 rpm. While mixing, a solution of 10 parts of molten 1,2-decanediol (Aldrich Chemical Co.) was added with the aid of a spray tip syringe over 5 minutes. This yielded 1.0 wt.% 1,2-decanediol on the HySorb^{®} T6600.

### Example 2

The procedure of Example 1 was repeated with a solution of 5 parts of molten 1,2-decanediol instead of 10 parts. This yielded 0.5 wt.% 1,2-decanediol on the HySorb^{®} T6600.

### Example 3

The procedure of Example 1 was repeated with a solution of 2.5 parts of molten 1,2-decanediol instead of 10 parts. This yielded 0.25 wt.% 1,2-decanediol on the HySorb^{®} T6600.

### Example 4

The procedure of Example 1 was repeated with a solution of 34.4 parts of cetrimonium chloride (29% solution in water, MakingCosmetics Inc.) instead of the 1,2-decediol solution. This yielded a total of 1 wt.% pure cetrimonium chloride on the HySorb^{®} T6600 infused with 0.35 wt.% Compound A.

The procedure of Example 1 was repeated with a solution of 17.2 parts of cetrimonium chloride (29% solution in water, MakingCosmetics Inc.) instead of the 1,2-decanediol solution. This yielded a total of 0.5 wt.% pure cetrimonium chloride on the HySorb^{®} T6600 infused with 0.35 wt.% Compound A.

### Example 6

The procedure of Example 1 was repeated with a solution of 8.6 parts of cetrimonium chloride (29% solution in water, MakingCosmetics Inc.) instead of the 1,2-decanediol solution. This yielded a total of 0.25 wt.% pure cetrimonium chloride on the HySorb^{®} T6600 infused with 0.35 wt.% Compound A.

Results of the examples are shown in Table 2. The ammonia BTT corresponds to room temperature incubation.

**Table 2: Comparison of results of inventive examples with comparative examples**

| **Sample** | **Compound A (wt.%)** | **Cetrimonium chloride (wt.%)** | **CRC (g/g)** | **AAP (g/g)** | **NH₃ BTT (hours)** |
|---|---|---|---|---|---|
| Comparative Example 5 | 0.35 | 0 | 33.9 | 23.7 | 19.0 |
| Comparative Example 4 | 0 | 0.5 | 36.2 | 20.1 | 49.7 |
| | | | | **Summation:** | 68.7 |
| Example 4 | 0.35 | 1 | 34.7 | 21.1 | Never started >70 |
| Example 5 | **0.35** | **0.5** | **35.3** | **22.3** | **Never started >72** |
| Example 6 | **0.35** | **0.25** | **35.2** | **22.8** | **Never started >72** |
| | | | | | |

| | **Compound A (wt.%)** | **1,2-decanediol (wt. %)** | **CRC (g/g)** | **AAP (g/g)** | **NH₃ BTT (hours)** |
|---|---|---|---|---|---|
| Comparative Example 5 | 0.35 | 0 | 33.9 | 23.7 | 19.0 |
| Comparative Example 1 | 0 | 0.5 | 36.0 | 19.9 | 18.0 |
| | | | | **Summation** | 37.0 |
| Example 1 | 0.35 | 1 | 36.4 | 17.9 | Never started >70 |
| Example 2 | 0.35 | 0.5 | 35.2 | 22.5 | 49.0 |
| Example 3 | 0.35 | 0.25 | 35.4 | 23.0 | 43.5 |

As indicated in Table 2, ammonia BTT exceeded 40 hours for each of the inventive examples. Comparative Examples 1, 5, and 6 resulted in an ammonia BTT of less than 20 hours at room temperature, and none of the Comparative Examples resulted in an ammonia BTT that exceeded 50 hours at room temperature.

Examples 1-6 indicate that high ammonia BTT is achievable without AAP falling below 20 g/g by using reduced amounts of auxiliary odor control agent in combination with primary odor control agent. In each of Examples 1-6, Compound A was used as the primary odor control agent, and was fixed at 0.35 wt.%. Cetrimonium chloride and 1,2-decanediol were used as different auxiliary odor control agents and were present at variable concentrations.

In Examples 1-3, 1,2-decanediol was varied from 1 wt.% in Example 1 down to 0.25 wt.% in Example 3. Example 1 exhibited an ammonia BTT that exceeded 70 hours; however, the AAP was below 20 g/g, which was due to the relatively high loading of 1,2-decanediol. When the 1,2-decanediol was reduced, ammonia BTT was reduced to the range of 40-50 hours; the AAP remained at above 20 g/g for both, which is preferable.

Examples 4-6 had significantly improved performance over the Comparative Examples. Here, the cetrimonium chloride concentration was varied from 1 wt.% down to 0.25 wt.%. The ammonia BTT and AAP performance were consistent for all three Examples, with ammonia BTT exceeding 70 hours, and AAP exceeding 20 g/g.

Examples 2 and 5 further indicate a synergistic effect on ammonia BTT when using primary and auxiliary odor control agents. Example 2 (0.35 wt.% Compound A and 0.5 wt.% 1,2-decanediol) resulted in an ammonia BTT of 49 hours, which was greater than each ammonia BTT for Comparative Example 1 (0.5 wt.% 1,2-decanediol) and Comparative Example 5 (0.35 wt.% Compound A) and the summations thereof (37 hours). Example 5 (0.35 wt.% Compound A and 0.5 wt.% cetrimonium chloride) resulted in an ammonia BTT of greater than 72 hours, which was greater than each ammonia BTT for Comparative Example 4 (0.5 wt.% cetrimonium chloride) and Comparative Example 5 (0.35 wt.% Compound A) and the summation thereof (68.7 hours).

### Comparative Example 8

A fluff pad rectangular sheet was prepared by dry mixing HySorb^{®} 6600 superabsorbent particles with a fluff material made from cellulose fibers. A polypropylene nonwoven top sheet was placed on the fluff pad sheet and compressed, and the assembly (which had a mass of about 99 g) was cut into several approximately 5.08-cm- diameter (2-inch-diameter) circular pads.

The pads included HySorb^{®} 6600 that was untreated with any odor control agents. The HySorb^{®} 6600 in each pad was present at about 20.2 wt.% based on a weight of the pad. The ammonia BTT was measured during incubation at 35 °C.

### Comparative Example 9

The procedure of Comparative Example 8 was repeated except that a 29% w/w solution of cetrimonium chloride was sprayed onto the HySorb^{®} 6600 prior to cutting the 99 g assembly into circular pads to deposit a total of 0.853 g cetrimonium chloride. This yielded cetrimonium chloride present at 0.25 wt.% based on a weight of the pad.

### Comparative Example 10

The procedure of Comparative Example 8 was repeated except that a HySorb^{®} 6600-Compound A infused sample was used with no additional treatment. This yielded Compound A present at 0.07 wt.% based on a weight of the pad.

Additional inventive examples are now described.

### Example 7

The procedure of Comparative Example 8 was repeated, except that a 29% w/w solution of cetrimonium chloride was sprayed onto a HySorb^{®} 6600-Compound A infused sample prior to cutting the 99 g assembly into circular pads to deposit a total of 0.853 g cetrimonium chloride. This yielded a total of 0.25 wt.% cetrimonium chloride and 0.07 wt.% Compound A based on a weight of the pad.

### Example 8 (not part of the invention)

The procedure of Comparative Example 8 was repeated, except that HySorb^{®} 6600 superadsorbent particles infused with Compound A and zinc peroxide were used. This yielded a total of 0.07 wt.% Compound A and 0.06 wt.% zinc peroxide based on a weight of the pad.

### Example 9

The procedure of Comparative Example 8 was repeated, except that a 29% w/w solution of cetrimonium chloride was sprayed onto HySorb^{®} 6600 superadsorbent particles infused with Compound A and zinc peroxide prior to cutting the 99 g assembly into circular pads to deposit a total of 0.853 g cetrimonium chloride. This yielded a total of 0.07 wt.% Compound A, 0.25 wt.% cetrimonium chloride, and 0.06 wt.% zinc peroxide based on a weight of the pad.

**Table 3: Comparison of inventive examples of two- and three-agent systems with comparative examples**

| **Sample** | **Compound A (wt.%)** | **Cetrimonium chloride (wt.%)** | **ZnO₂ (wt.%)** | **NH₃ BTT (hours)** |
|---|---|---|---|---|
| Comparative Example 8 | 0 | 0 | 0 | 6.3 |
| Comparative Example 9 | 0 | 0.25 | 0 | 7.0 |
| Comparative Example 10 | 0.07 | 0 | 0 | 7.7 |
| Example 7 | 0.07 | 0.25 | 0 | 9.0 |
| Example 8 | 0.07 | 0 | 0.06 | 10.0 |
| Example 9 | 0.07 | 0.25 | 0.06 | 13.0 |

As indicated in Table 3, Examples 7 and 8, which included two odor control agents, resulted in a longer ammonia BTT compared to Comparative Examples 8-10. Example 9, which included three odor control agents, resulted in an even longer ammonia BTT than Examples 7 and 8.

The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is intended to present concepts in a concrete fashion.

As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. In addition, the articles "a" and "an" as used in this application and the appended claims should generally be construed to mean "one or more" unless specified otherwise or clear from context to be directed to a singular form.

Reference throughout this specification to "an embodiment", "certain embodiments", or "one embodiment" means that a particular feature, structure, or characteristic described in connection with the embodiment is included in at least one embodiment. Thus, the appearances of the phrase "an embodiment", "certain embodiments", or "one embodiment" in various places throughout this specification are not necessarily all referring to the same embodiment, and such references mean "at least one".

## Claims

1. An odor-inhibiting composition comprising:
water-absorbing polymer particles;
a first odor control agent comprising a chelation agent; and
a second odor control agent,
wherein the chelation agent is selected from a group consisting of: ethylenediaminetetraacetic acid (EDTA), nitrilotris(methylene)triphosphonic acid, sodium triphosphate pentabasic, N-carboxymethylated polyethyleneimine, and 1-hydroxyethane 1,1-diphosphonic acid, or salts thereof; and
the second odor control agent is a membrane lysing compound selected from a group consisting of: 1,2-decanediol, cetrimonium chloride, cocamidopropyl betaine, poly(t-butylaminoethylmethacrylate), behentrimonium, and 1,2-pentanediol.

2. The composition of claim 1, wherein the first odor control agent is present from about 0.25 wt.% to about 1.0 wt.% based on a total weight of the water-absorbing polymer particles, and wherein the second odor control agent is present from about 0.25 wt.% to about 2.0 wt.% based on the total weight of the water-absorbing polymer particles.

3. The composition of any of the preceding claims, wherein the water-absorbing polymer particles comprise crosslinked polyacrylic acid.

4. The composition of any of the preceding claims, wherein the water-absorbing polymer particles are polymerized in the presence of one or more of the first odor control agent or the second odor control agent.

5. The composition of any of the preceding claims, wherein the second odor control agent comprises 1,2-decanediol or cetrimonium chloride.

6. The composition of any of the preceding claims, wherein the composition comprises a third odor control agent, wherein the third odor control agent is a different compound than the first odor control agent and the second odor control agent.

7. The composition of claim 6, wherein the third odor control agent comprises zinc peroxide.

8. A method of producing an odor-inhibiting composition, the method comprising:
infusing water-absorbing polymer particles with a first odor control agent comprising a chelation agent; and
mixing the infused water-absorbing polymer particles with a second odor control agent, wherein the chelation agent is selected from a group consisting of: ethylenediaminetetraacetic acid (EDTA), nitrilotris(methylene)triphosphonic acid, sodium triphosphate pentabasic, N-carboxymethylated polyethyleneimine, and 1-hydroxyethane 1,1-diphosphonic acid, or salts thereof; and
the second odor control agent is a membrane lysing compound selected from a group consisting of: 1,2-decanediol, cetrimonium chloride, cocamidopropyl betaine, poly(t-butylaminoethylmethacrylate), behentrimonium, and 1,2-pentanediol.

9. The method of claim 8, wherein the first odor control agent is present from about 0.25 wt.% to about 1.0 wt.% based on a total weight of the water-absorbing polymer particles, and wherein the second odor control agent is present from about 0.25 wt.% to about 2.0 wt.% based on the total weight of the water-absorbing polymer particles.

10. The method of any of either claim 8 or claim 9, wherein infusing water-absorbing polymer particles with the first odor control agent comprises polymerizing a monomer solution in the presence of the first odor control agent.

11. The method of any of claims 8-10, wherein the second odor control agent comprises 1,2-decanediol or cetrimonium chloride.

12. A hygienic article comprising an odor-inhibiting composition, the composition comprising:
water-absorbing polymer particles;
a first odor control agent comprising a chelation agent; and
a second odor control agent,
wherein the chelation agent is selected from a group consisting of: ethylenediaminetetraacetic acid (EDTA), nitrilotris(methylene)triphosphonic acid, sodium triphosphate pentabasic, N-carboxymethylated polyethyleneimine, and 1-hydroxyethane 1,1-diphosphonic acid, or salts thereof; and
the second odor control agent is a membrane lysing compound selected from a group consisting of: 1,2-decanediol, cetrimonium chloride, cocamidopropyl betaine, poly(t-butylaminoethylmethacrylate), behentrimonium, and 1,2-pentanediol.

13. The hygienic article of claim 12, wherein the first odor control agent is present from about 0.25 wt.% to about 1.0 wt.% based on a total weight of the water-absorbing polymer particles, and wherein the second odor control agent is present from about 0.25 wt.% to about 2.0 wt.% based on the total weight of the water-absorbing polymer particles.

14. The hygienic article of either claim 12 or claim 13, wherein the hygienic article is selected from a group consisting of: a diaper, a diaper pad, a feminine hygienic article, an incontinence article, a bed pad, a nursing pad, animal litter, an animal training pad, and a pet diaper.

15. The odor-inhibiting composition of claim 1, comprising:
water absorbing particles comprising crosslinked polyacrylic acid; cetrimonium chloride; and
1-hydroxyethane 1,1-diphosphonic acid or salts thereof.

## Patentansprüche

1. Geruchshemmende Zusammensetzung, umfassend:
wasserabsorbierende Polymerteilchen;
ein erstes Geruchsunterdrückungsmittel, umfassend einen Chelatbildner; und
ein zweites Geruchsunterdrückungsmittel,
wobei der Chelatbildner aus einer Gruppe ausgewählt ist, bestehend aus: Ethylendiamintetraessigsäure (EDTA), Nitrilotris(methylen)triphosphonsäure, Natriumtriphosphat pentabasisch, N-carboxymethyliertem Polyethylenimin und 1-Hydroxyethan-1,1-diphosphonsäure, oder Salzen davon; und
wobei das zweite Geruchsunterdrückungsmittel eine Membranlyseverbindung ist, die aus einer Gruppe ausgewählt ist, bestehend aus: 1,2-Decandiol, Cetrimoniumchlorid, Cocamidopropylbetain, Poly(t-butylaminethylmethacrylat), Behentrimonium und 1,2-Pentandiol.

2. Zusammensetzung nach Anspruch 1, wobei das erste Geruchsunterdrückungsmittel von etwa zu 0,25 Gew.-% bis etwa 1,0 Gew.-%, bezogen auf ein Gesamtgewicht der wasserabsorbierenden Polymerteilchen, vorhanden ist und wobei das zweite Geruchsunterdrückungsmittel von etwa zu 0,25 Gew.-% bis etwa 2,0 Gew.-%, bezogen auf das Gesamtgewicht der wasserabsorbierenden Polymerteilchen, vorhanden ist.

3. Zusammensetzung nach einem der vorstehenden Ansprüche, wobei die wasserabsorbierenden Polymerteilchen eine vernetzte Polyacrylsäure umfassen.

4. Zusammensetzung nach einem der vorstehenden Ansprüche, wobei die wasserabsorbierenden Polymerteilchen unter Einwirkung von einem oder mehreren des ersten Geruchsunterdrückungsmittels oder des zweiten Geruchsunterdrückungsmittels polymerisiert werden.

5. Zusammensetzung nach einem der vorstehenden Ansprüche, wobei das zweite Geruchsunterdrückungsmittel 1,2-Decandiol oder Cetrimoniumchlorid umfasst.

6. Zusammensetzung nach einem der vorstehenden Ansprüche, wobei die Zusammensetzung ein drittes Geruchsunterdrückungsmittel umfasst, wobei das dritte Geruchsunterdrückungsmittel eine andere Verbindung als das erste Geruchsunterdrückungsmittel und das zweite Geruchsunterdrückungsmittel ist.

7. Zusammensetzung nach Anspruch 6, wobei das dritte Geruchsunterdrückungsmittel Zinkperoxid umfasst.

8. Verfahren zum Herstellen einer geruchshemmenden Zusammensetzung, das Verfahren umfassend:
Infundieren von wasserabsorbierenden Polymerteilchen mit einem ersten Geruchsunterdrückungsmittel, das einen Chelatbildner umfasst; und
Mischen der infundierten wasserabsorbierenden Polymerteilchen mit einem zweiten Geruchsunterdrückungsmittel, wobei der Chelatbildner aus einer Gruppe ausgewählt ist, die aus Folgendem besteht: Ethylendiamintetraessigsäure (EDTA), Nitrilotris(methylen)triphosphonsäure, Natriumtriphosphat pentabasisch, N-carboxymethylierten Polyethylenimin und 1-Hydroxyethan-1,1-diphosphonsäure, oder Salzen davon; und
das zweite Geruchsunterdrückungsmittel eine Membranlyseverbindung ist, die aus einer Gruppe ausgewählt ist, bestehend aus: 1,2-Decandiol, Cetrimoniumchlorid, Cocamidopropylbetain, Poly(t-butylaminethylmethacrylat), Behentrimonium und 1,2-Pentandiol.

9. Verfahren nach Anspruch 8, wobei das erste Geruchsunterdrückungsmittel von etwa zu 0,25 Gew.-% bis etwa 1,0 Gew.-%, bezogen auf ein Gesamtgewicht der wasserabsorbierenden Polymerteilchen, vorliegt und wobei das zweite Geruchsunterdrückungsmittel von etwa zu 0,25 Gew.-% bis etwa 2,0 Gew.-%, bezogen auf das Gesamtgewicht der wasserabsorbierenden Polymerteilchen, vorliegt.

10. Verfahren nach einem der Ansprüche 8 oder 9, wobei das Infundieren von wasserabsorbierenden Polymerteilchen mit dem ersten
Geruchsunterdrückungsmittel, das ein Polymerisieren einer Monomerlösung unter Einwirkung des ersten Geruchsunterdrückungsmittels, umfasst.

11. Verfahren nach einem der Ansprüche 8 bis 10, wobei das zweite Geruchsunterdrückungsmittel 1,2-Decandiol oder Cetrimoniumchlorid umfasst.

12. Hygieneartikel, umfassend eine geruchshemmende Zusammensetzung, die Zusammensetzung umfassend:
wasserabsorbierende Polymerteilchen;
ein erstes Geruchsunterdrückungsmittel, umfassend einen Chelatbildner; und
ein zweites Geruchsunterdrückungsmittel,
wobei der Chelatbildner aus einer Gruppe ausgewählt ist, bestehend aus: Ethylendiamintetraessigsäure (EDTA), Nitrilotris(methylen)triphosphonsäure, Natriumtriphosphat pentabasisch, N-carboxymethylierten Polyethylenimin und 1-Hydroxyethan-1,1-diphosphonsäure, oder Salzen davon; und
das zweite Geruchsunterdrückungsmittel eine Membranlyseverbindung ist, die aus einer Gruppe ausgewählt ist, bestehend aus: 1,2-Decandiol, Cetrimoniumchlorid, Cocamidopropylbetain, Poly(t-butylaminethylmethacrylat), Behentrimonium und 1,2-Pentandiol.

13. Hygieneartikel nach Anspruch 12, wobei das erste Geruchsunterdrückungsmittel von etwa zu 0,25 Gew.-% bis etwa 1,0 Gew.-%, bezogen auf ein Gesamtgewicht der wasserabsorbierenden Polymerteilchen, vorhanden ist und wobei das zweite Geruchsunterdrückungsmittel von etwa zu 0,25 Gew.-% bis etwa 2,0 Gew.-%, bezogen auf das Gesamtgewicht der wasserabsorbierenden Polymerteilchen, vorhanden ist.

14. Hygieneartikel entweder nach Anspruch 12 oder 13, wobei der Hygieneartikel aus einer Gruppe ausgewählt ist, bestehend aus: einer Windel, einer Windeleinlage, einem Damenhygieneartikel, einem Inkontinenzartikel, einer Betteinlage, einer Stilleinlage, Tierstreu, einer Tiertrainingseinlage und einer Haustierwindel.

15. Geruchshemmende Zusammensetzung nach Anspruch 1, umfassend:
Wasser absorbierende Teilchen, umfassend die vernetzte Polyacrylsäure; Cetrimoniumchlorid; und
1-Hydroxyethan-1,1-diphosphonsäure, oder Salze davon.

## Revendications

1. Composition inhibitrice d'odeurs comprenant :
des particules polymères absorbant l'eau ;
un premier agent de contrôle d'odeurs comprenant un agent de chélation ; et
un deuxième agent de contrôle d'odeurs,
l'agent de chélation étant choisi dans un groupe constitué par : acide éthylènediamine-tétracétique (EDTA), acide nitrilotris(méthylène)triphosphonique, triphosphate de sodium pentabasique, polyéthylèneimine N-carboxyméthylée et acide 1-hydroxyéthane 1,1-diphosphonique, ou sels de ceux-ci ; et
le deuxième agent de contrôle d'odeurs étant un composé de lyse de membrane choisi dans un groupe constitué par : 1,2-décanediol, chlorure de cétrimonium, cocamidopropyl-bétaïne, poly(t-butylaminoéthylméthacrylate), béhentrimonium et 1,2-pentanediol.

2. Composition selon la revendication 1, dans laquelle le premier agent de contrôle d'odeurs est présent à raison d'environ 0,25 % en poids à environ 1,0 % en poids en fonction d'un poids total des particules polymères absorbant l'eau, et dans laquelle le deuxième agent de contrôle d'odeurs est présent à raison d'environ 0,25 % en poids à environ 2,0 % en poids en fonction du poids total des particules polymères absorbant l'eau.

3. Composition selon l'une quelconque des revendications précédentes, dans laquelle les particules polymères absorbant l'eau comprennent de l'acide polyacrylique réticulé.

4. Composition selon l'une quelconque des revendications précédentes, dans laquelle les particules polymères absorbant l'eau sont polymérisées en présence d'un ou plusieurs parmi le premier agent de contrôle d'odeurs ou le deuxième agent de contrôle d'odeurs.

5. Composition selon l'une quelconque des revendications précédentes, dans laquelle le deuxième agent de contrôle d'odeurs comprend 1,2-décanediol ou chlorure de cétrimonium.

6. Composition selon l'une quelconque des revendications précédentes, la composition comprenant un troisième agent de contrôle d'odeurs, le troisième agent de contrôle d'odeurs étant un composé différent du premier agent de contrôle d'odeurs et du deuxième agent de contrôle d'odeurs.

7. Composition selon la revendication 6, dans laquelle le troisième agent de contrôle d'odeurs comprend du peroxyde de zinc.

8. Procédé de production d'une composition inhibitrice d'odeurs, le procédé comprenant :
l'infusion de particules polymères absorbant l'eau avec un premier agent de contrôle d'odeurs comprenant un agent de chélation ; et
le mélange des particules polymères absorbant l'eau infusées avec un deuxième agent de contrôle d'odeurs, l'agent de chélation étant choisi dans un groupe constitué par : acide éthylènediamine-tétracétique (EDTA), acide nitrilotris(méthylène)triphosphonique, triphosphate de sodium pentabasique, polyéthylèneimine N-carboxyméthylée et acide 1-hydroxyéthane 1,1-diphosphonique, ou sels de ceux-ci ; et
le deuxième agent de contrôle d'odeurs est un composé de lyse de membrane choisi dans un groupe constitué par : 1,2-décanediol, chlorure de cétrimonium, cocamidopropyl-bétaïne, poly(t-butylaminoéthylméthacrylate), béhentrimonium et 1,2-pentanediol.

9. Procédé selon la revendication 8, dans lequel le premier agent de contrôle d'odeurs est présent à raison d'environ 0,25 % en poids à environ 1,0 % en poids en fonction d'un poids total des particules polymères absorbant l'eau, et dans laquelle le deuxième agent de contrôle d'odeurs est présent à raison d'environ 0,25 % en poids à environ 2,0 % en poids en fonction du poids total des particules polymères absorbant l'eau.

10. Procédé selon l'une quelconque parmi la revendication 8 ou la revendication 9, dans lequel l'infusion de particules polymères absorbant l'eau avec le premier agent de contrôle des odeurs comprend la polymérisation d'une solution de monomère en présence du premier agent de contrôle d'odeurs.

11. Procédé selon l'une quelconque des revendications 8 à 10, dans lequel le deuxième agent de contrôle d'odeurs comprend 1,2-décanediol ou chlorure de cétrimonium.

12. Article hygiénique comprenant une composition inhibitrice d'odeurs, la composition comprenant :
des particules polymères absorbant l'eau ;
un premier agent de contrôle d'odeurs comprenant un agent de chélation ; et
un deuxième agent de contrôle d'odeurs,
l'agent de chélation étant choisi dans un groupe constitué par : acide éthylènediamine-tétracétique (EDTA), acide nitrilotris(méthylène)triphosphonique, triphosphate de sodium pentabasique, polyéthylèneimine N-carboxyméthylée et acide 1-hydroxyéthane 1,1-diphosphonique, ou sels de ceux-ci ; et
le deuxième agent de contrôle d'odeurs est un composé de lyse de membrane choisi dans un groupe constitué par : 1,2-décanediol, chlorure de cétrimonium, cocamidopropyl-bétaïne, poly(t-butylaminoéthylméthacrylate), béhentrimonium et 1,2-pentanediol.

13. Article hygiénique selon la revendication 12, dans lequel le premier agent de contrôle d'odeurs est présent à raison d'environ 0,25 % en poids à environ 1,0 % en poids en fonction d'un poids total des particules polymères absorbant l'eau, et dans lequel le deuxième agent de contrôle d'odeurs est présent à raison d'environ 0,25 % en poids à environ 2,0 % en poids en fonction du poids total des particules polymères absorbant l'eau.

14. Article hygiénique selon la revendication 12 ou la revendication 13, l'article hygiénique étant choisi dans un groupe constitué par : une couche, un tapis à langer, un article hygiénique féminin, un article pour l'incontinence, une alèse, un coussinet d'allaitement, une litière pour animaux, un tapis d'éducation pour animaux et une couche pour animaux de compagnie.

15. Composition inhibitrice d'odeurs selon la revendication 1, comprenant :
des particules absorbant l'eau comprenant de l'acide polyacrylique réticulé ;
du chlorure de cétrimonium ; et
de l'acide 1-hydroxyéthane 1,1-diphosphonique ou sels de celui-ci.
